# EUROPEAN PATENT SPECIFICATION

(11) **EP 3 202 374 B1**
(45) Date of publication and mention of the grant of the patent: **30.10.2019**
(21) Application number: 15846643.3
(22) Date of filing: 28.09.2015
(51) Int. Cl.: A61F 5/01, A61F 5/02

(54) **ORTHOSIS**
ORTHESE
ORTHÈSE

(30) Priority: 29.09.2014 JP 2014198623
(43) Date of publication of application: 09.08.2017
(73) Proprietor: The University of Electro-Communications, Tokyo 182-8585 (JP); Kikuchi Seisakusho Co., Ltd., Tokyo 192-0152 (JP); National University Corporation University Of Toyama, Toyama-shi, Toyama 930-8555 (JP); TSS Co., Ltd., Shinagawa-ku Tokyo 1420041 (JP)
(72) Inventor: KAJIMOTO, Hiroyuki, Chofu-shi Tokyo 182-8585 (JP); SATO, Michi, Chofu-shi, Tokyo 182-8585 (JP); IGUCHI, Takeyoshi, Hachioji-shi Tokyo 192-0152 (JP); ASAHI, Takashi, Toyama-shi Toyama 930-08555 (JP)
(74) Representative: Weickmann & Weickmann PartmbB
(86) International application number: PCT/JP2015/077313
(87) International publication number: WO 2016/052400

(56) References cited:
- EP-A1- 2 601 913
- WO-A1-2012/017889
- JP-A- S4 740 884
- JP-A- H07 308 333
- JP-A- 2011 147 749
- JP-A- 2014 000 185
- JP-B2- 5 552 844
- US-B1- 6 423 019
- US-B1- 6 592 536
- MICHI SATO ET AL: "Development of a head rotation interface by using Hanger Reflex", ROBOT AND HUMAN INTERACTIVE COMMUNICATION, 2009. RO-MAN 2009. THE 18TH IEEE INTERNATIONAL SYMPOSIUM ON, IEEE, PISCATAWAY, NJ, USA, 27 September 2009 (2009-09-27), pages 534-538, XP031563695, ISBN: 978-1-4244-5081-7
- TAKASHI ASAHI ET AL: "Rate of Hanger Reflex Occurrence: Unexpected Head Rotation on Fronto-temporal Head Compression", NEUROLOGIA MEDICO-CHIRURGICA, vol. 55, no. 7, 1 January 2015 (2015-01-01), pages 587-591, XP055472992, ISSN: 0470-8105, DOI: 10.2176/nmc.oa.2014-0324

## Description

### Technical Field

The present invention relates to an orthosis that induces a rotational movement of the head.

### Background Art

In general, a disease called spasmodic torticollis has been known. Spasmodic torticollis is a type of local dystonia that causes involuntary movements, such as inclination, twist, and trembling of the head in any of horizontal and vertical directions. Spasmodic torticollis is also called cervical dystonia. It is said that about ten to thirty thousand spasmodic torticollis patients are in Japan. Unfortunately, the cause of spasmodic torticollis has not been clarified.

Physical therapy, pharmacotherapy, and surgical treatment have been practiced as treatment for spasmodic torticollis. The physical therapy includes relaxation and biofeedback through autogenic training. The pharmacotherapy includes injection of botulinus toxin. The surgical treatment includes deep brain stimulation, selective peripheral denervation, and accessory nerve decompression.

Besides the treatments described above, to resolve spasmodic torticollis, an orthosis utilizing the hanger reflex has been known (e.g., Patent Literature 1). The orthosis described in Patent Literature 1 has a substantially elliptic shape and elasticity. The length of the minor axis of the orthosis corresponds to the width of the head. Consequently, when the orthosis is worn with being fitted to temporal regions, the orthosis is in contact with the temporal regions or therearound. In this case, opposite pressures on a straight line in directions from the right and left temporal regions of the head toward the center are applied, and the orthosis can be worn in a stable state. When such an orthosis is worn in a deviating manner, head rotation is induced.

The mechanism that induces such head rotation has not been clarified. According to a possible hypothesis, a perception (illusion) of an external force that tends to rotate the head may be given.

More specifically, before the orthosis described in Patent Literature 1 is worn in the deviating manner, the opposite pressures on the one straight line in the directions at the temporal regions toward the inside are applied. Meanwhile, deviation of the orthosis causes the positions where the orthosis is in contact with the head to deviate. Furthermore, as the orthosis has the substantially elliptic shape, the state is achieved where the pressures to the head are not opposite on the single straight line. The orthosis in this state is accompanied by skin on the head where the orthosis is in contact, thus causing a force of returning to the original stable state. Here, the slack of skin on the head causes skin deformation at portions of the orthosis where this orthosis is in contact.

There is a hypothesis that the skin deformation caused by returning the deviating state to the original state provides a wearer with a perception (illusion) of an external force for turning the head, thereby inducing a head rotation.

Such an orthosis described in Patent Literature 1 contributes to alleviation of the symptom of spasmodic torticollis by inducing a rotational movement in a direction opposite to the direction of the rotational movement of the head which is the symptom of a spasmodic torticollis patient in conformity with the symptom of the spasmodic torticollis patient. More specifically, a patient having rotation in the clockwise direction wears the orthosis so as to induce a rotational movement in the counterclockwise direction, thereby allowing the rotation of the patient to be alleviated.

### Citation List

### Patent Literature

JP 2011 147 749 A as well as JP 5552844 B2 relates to an orthosis to suppress rotation motion of the head due to spasmodic torticollis, disclosing the preamble of claim 1.

EP 2 601 913 A1 discloses a posture correcting tool with a simple structure.

### Summary of Invention

### Technical Problem

Unfortunately, the orthosis described in JP 5552844 B2 has a problem of difficulty in selling as a general product.

More specifically, the orthosis described in Patent Literature 1 has the shape that fits the temporal regions of the wearer. Accordingly, the size of the orthosis is determined in conformity with the head size of the wearer. It is therefore ideal that the size of the orthosis is determined in a custom-made manner in conformity with the head size of each individual wearer. However, creation and selling of orthoses in a custom-made manner lacks general versatility, gives burdensomeness to the wearer, and is not preferable also in view of cost.

Through use of the elasticity of the orthoses, the orthoses can be designed to support various head sizes of wearers, standardized into several sizes of orthoses, and offered in a product lineup. However, to allow many wearers to wear the orthoses effectively, orthoses having a variety of sizes are required to be sold. This requirement is not preferable in view of size management and cost. Furthermore, there is another problem of giving the wearers burdensomeness of searching for the size of the orthosis that can fit the own head size.

In view of such situations, development of an orthosis is expected that has advantageous effects analogous to those of the orthosis described in Patent Literature 1 and is adjustable to the head size of a wearer.

The present invention thus has an object to provide an orthosis that is adjustable to the head size of a wearer and induces a rotational movement of the head.

### Solution to Problem

This object is achieved by an orthosis according claim 1.

Preferably, a ratio of change in the substantially elliptic shape in the direction of the minor axis due to adjustment of the adjuster is larger than a ratio of change in the substantially elliptic shape in a direction of the major axis.

Preferably, when the adjustment portions are adjusted by the adjuster so that the length of the minor axis of the substantially elliptic shape can be adapted to correspond to the width of the head, the connecting member is formed at a position which does not press the head.

Preferably, the connecting member has a belt shape, and one of a hook and a loop of a surface fastener is provided on an inner side of the connecting member, and the other one of the hook and the loop of the surface fastener is provided on an outer side of the outer layer.

Preferably, the orthosis further includes supporters each having holes through which the connecting member is to be inserted and provided at opposite ends of the opening of the outer layer, wherein the connecting member is adapted to be inserted through the hole, and the surface fastener on the inner side of the connecting member is connected to the surface fastener on the outer side of the outer layer.

Preferably, the connecting member is formed of a first connecting member and a second connecting member which are respectively connected to opposite ends of the opening, and a fixing section which adjusts and fixes a position where the first connecting member and the second connecting member are connected to each other, and according to the position of fixation by the fixing section, lengths from the opposite ends of the opening to the fixing section are adjusted to adjust the adjustment portions so that the length of the minor axis of the substantially elliptic shape can be adapted to correspond to the width of the head.

Preferably, orthosis further includes a detachable section which is detachably attached to an inner side of the buffer and is in contact with the head, wherein the detachable section is formed of a member which can be adapted to transmit pressure on the head to the buffer.

### Advantageous Effects of Invention

The present invention can provide an orthosis that is adjustable to the head size of a wearer and induces a rotational movement of the head.

### Brief Description of Drawings

FIG. 1 is a diagram of an orthosis that is an orthosis according to an embodiment of the present invention which corresponds to a wearer having a wide head width.
FIG. 2 is a diagram of an orthosis that is an orthosis according to the embodiment of the present invention which corresponds to a wearer having a narrow head width.
FIG. 3 is a diagram illustrating an adjuster of the orthosis according to the embodiment of the present invention.
FIG. 4A and FIG. 4B are diagrams illustrating a supporter of the orthosis according to the embodiment of the present invention.
FIG. 5A and FIG. 5B are top views of a state where a wearer having a wide head width wears the orthosis according to the embodiment of the present invention.
FIG. 6A and FIG. 6B are top views of a state where a wearer having a narrow head width wears the orthosis according to the embodiment of the present invention.
FIG. 7A and FIG. 7B are diagrams illustrating formation of pressers of the orthosis according to the embodiment of the present invention.
FIG. 8A, FIG. 8B, and FIG. 8C are diagrams illustrating pressers that induce right and left (clockwise and counterclockwise) rotations by the orthosis according to the embodiment of the present invention.
FIG. 9A, FIG. 9B, and FIG. 9C are diagrams illustrating pressers that induce a lateroversion rotation by the orthosis according to the embodiment of the present invention.
FIG. 10A, FIG. 10B, and FIG. 10C are diagrams illustrating pressers that induce a retroflexion rotation by the orthosis according to the embodiment of the present invention.
FIG. 11A, FIG. 11B, and FIG. 11C are diagrams illustrating pressers that induce a rotation in which lateroversion and retroflexion rotations are combined, by the orthosis according to an embodiment of the present invention.
FIG. 12 is a diagram illustrating an orthosis according to a first variation of the present invention (first example).
FIG. 13 is a diagram illustrating an orthosis according to the first variation of the present invention (second example).
FIG. 14 is a diagram illustrating an orthosis according to the first variation of the present invention (third example).
FIG. 15 is a diagram illustrating an orthosis according to the first variation of the present invention (fourth example).
FIG. 16A and FIG. 16B are diagrams illustrating an orthosis according to a second variation of the present invention (first example).
FIG. 17 is a diagram illustrating an orthosis according to the second variation of the present invention (second example).

### Description of Embodiments

Next, referring to the drawings, embodiments of the present invention are described. In the following illustration in drawings, the same or similar elements are assigned the same or similar reference signs.

### (Embodiments)

Referring to FIG.s 1 to 7, an orthosis 1 according to an embodiment of the present invention is described.

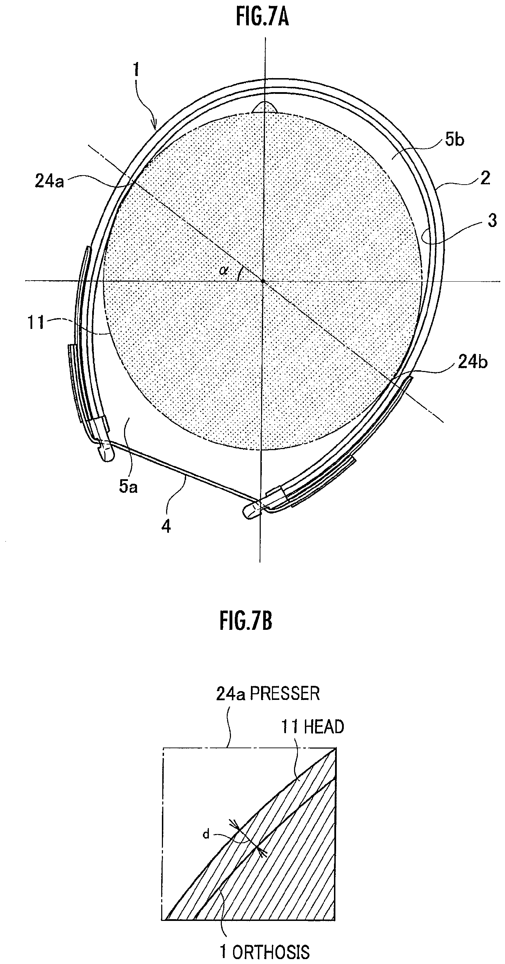

The orthosis 1 has a shape adjustable to the head. The orthosis 1 has a substantially elliptic shape, and is worn so as to be in contact with proximity to the temporal regions (temples) of a head 11 of the wearer. The orthosis 1 according to the embodiment of the present invention is worn in a state deviate from a state where the length of the minor axis of the substantially elliptic shape corresponds to the width of the head 11. In this case, through use of the elasticity of an outer layer 2, pressers which press predetermined sites on the head 11 with forces stronger than those before deviation are formed to induce a rotational movement of the head. In the embodiment of the present invention, as shown in FIG. 7, through use of the elasticity of the outer layer 2, pressers 24a and 24b which press at least two sites are formed.

The orthosis 1 according to the embodiment of the present invention is applicable as a therapeutic instrument for spasmodic torticollis, and specifically, can alleviate the symptom of rotatory spasmodic torticollis. The orthosis 1 can contribute not only to alleviation of spasmodic torticollis but also generally to alleviation of torticollis symptoms which indicate head abnormality and include symptoms of shoulder stiffness.

According to the embodiment of the present invention, the wearer wears the orthosis 1 so that the minor axis of the substantially elliptic shape can correspond to the line connecting the opposite temporal regions. Consequently, according to the embodiment of the present invention, "major axis direction" corresponds to the anteroposterior direction of the wearer when the wearer wears the orthosis 1 at the front, and "minor axis direction" corresponds to the lateral direction of the wearer when the wearer wears the orthosis 1 at the front.

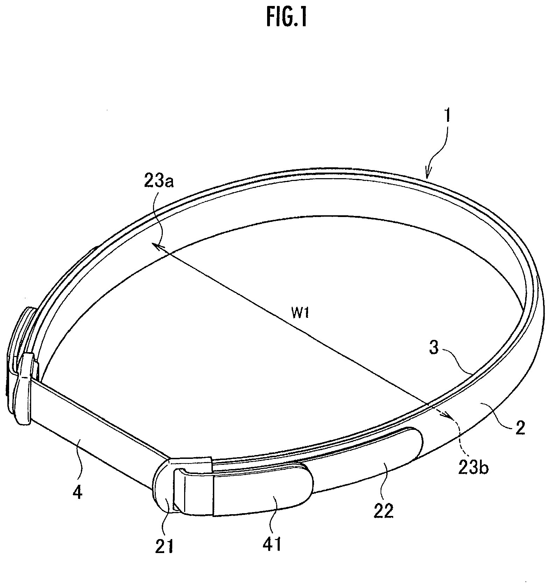

As shown in FIG. 1, the orthosis 1 comprises the outer layer 2, a buffer 3, and an adjuster 4. A case where the adjuster 4 has a belt shape in the embodiment of the present invention is described. As described in an after-mentioned first variation, the orthosis 1 according to the embodiment of the present invention is applicable also to a case where the adjuster 4 does not have the belt shape.

As shown in FIG.s 1 and 2, the orthosis 1 according to the embodiment of the present invention can adjust the length of the orthosis 1 in the minor axis direction of the substantially elliptic shape through the adjuster 4 in conformity with the width of the head 11 of the wearer.

### (Outer layer)

The outer layer 2 has an elasticity to an extent wearable on the head 11. The material of the outer layer 2 may be aluminum whose material surface has been anodized. In the case where the outer layer 2 is formed of the aluminum described above, this layer has a width to an extent of two centimeters and a thickness to an extent of two millimeters. In conformity with the degree of rotation to be induced and with material and the like, the width and thickness of the outer layer 2 are appropriately configured.

The outer layer 2 maintains the substantially elliptic shape. Here, the "substantially elliptic shape" means a shape formed by pressing opposite portions of a circle. The shape is not necessarily an algebraically representable ellipse. In the embodiment of the present invention, the outer layer 2 has a shape laid around the opposite temporal regions of the wearer. The outer layer 2 has a substantially C-shape formed by providing an opening at the front or rear in the major axis direction of the substantially elliptic shape. The case where the opening is provided at the rear of the wearer is described in this embodiment of the present invention. Alternatively, the opening may be provided at the front. As shown in FIG. 1, the opposite sides of the opening of the outer layer 2 are connected by a connecting member which forms the adjuster 4.

In the embodiment of the present invention, the outer layer 2 includes adjustment portions 23a and 23b which can be close to or apart from each other in the minor axis direction of the substantially elliptic shape.

In the embodiment of the present invention, as shown in FIG. 1, the adjustment portions 23a and 23b are provided in proximity to the minor axis of the orthosis 1. The adjustment portions 23a and 23b are arranged at portions which are closest to the proximity to the temporal regions of the head 11 of the wearer when the orthosis 1 is worn at the front of the wearer with the adjuster 4 being positioned at the occiput of the wearer. Subsequently, the adjuster 4 adjusts the positions of the adjustment portions 23a and 23b so that the adjustment portions 23a and 23b can be in contact with the proximity of the respective temporal regions of the wearer.

The positions of the adjustment portions 23a and 23b are adjusted so as to fit the shapes of the heads of various wearers with various sizes. The adjustable width of the adjustment portions 23a and 23b is appropriately set in conformity with the shapes of the heads of the wearers in a sales area of the orthosis 1. More specifically, according to discussion of the shapes of Japanese people, it has been known that at least 90% of Japanese people have head widths with errors ranging within 2.5 cm. Consequently, in the case of the orthosis 1 being sold in Japan, it is preferred that the distance between the adjustment portions 23a and 23b should have an adjustable width to an extent of 2.5 cm in order to fit the shapes of the heads of many Japanese people. The adjustable width is configured to be adjusted in conformity with the material of the outer layer 2 and the size of the opening.

When the adjustment portions 23a and 23b are herein adjusted by the adjuster 4 so that the length of the minor axis of the substantially elliptic shape can correspond to the width of the head 11, the connecting member that forms the adjuster 4 is arranged at a position where the head 11 is not pressed. In other words, the opposite ends of the opening of the outer layer 2 are provided to be extended to the occiput positions. On the other hand, when the opposite ends of the opening of the outer layer 2 are provided in the opposite temporal regions, the connecting member that connects the opposite ends sometimes presses the occiput strongly to prevent the pressers from being appropriately formed in the outer layer 2 in a certain case of the curved shape of the occiput of the head 11. Consequently, appropriate formation of the pressers 24a and 24b are realized by providing the opposite ends of the opening of the outer layer 2 so as to be elongated to the occiput positions to achieve situations where the connecting member does not press the head 11.

In the embodiment of the present invention, the case is described where the adjustment portions 23a and 23b are provided in proximity to the minor axis of the orthosis 1 and around the temporal regions of the head 11 as shown in FIG. 1 and the like. Only if the length of the orthosis 1 in the minor axis direction is adjustable to the width of the head 11, any configuration may be adopted. For example, the length of the orthosis 1 in the minor axis direction may be adjusted to the width of the head 11 by providing the adjustment portions 23a and 23b at positions which are apart from the temporal regions and may be positions at the rear of the temporal regions of the orthosis 1 and by causing the adjuster 4 to adjust the adjustment portions 23a and 23b.

Furthermore, in the embodiment of the present invention, the case is described where the two adjustment portions 23a and 23b are provided at bilaterally symmetric positions. However, the configuration is not limited thereto. The orthosis 1 may comprise at least three adjustment portions. The adjuster 4 may adjust these adjustment portions to adjust the length of the orthosis 1 in the minor axis direction to the width of the head 11. Alternatively, in a case where one of the right and left of the orthosis 1 is fixed, the adjustment portion may be provided at the other one which is not fixed. In this case, the adjuster 4 may adjust an adjustment portion (the one without fixation) so that the minor axis of the substantially elliptic shape can correspond to the width of the head 11.

### (Buffer)

The buffer 3 is provided on the inner periphery of the outer layer 2, and buffers the predetermined sites on the head 11. The predetermined sites of the head 11 are the front of a temporal region and the rear of the opposite temporal region, the upper forehead and lower occiput, the lower forehead and upper occiput, the upper part of a temporal region and a lower part of the opposite temporal region, the upper left forehead and the lower right occiput, the upper right forehead and lower left occiput, lower left forehead and upper right occiput, or the lower right forehead and upper left occiput.

The buffer 3 is made of a material which causes extremely strong frictions when the buffer 3 has a frictional force to an extent capable of rotating the orthosis 1 and strong contact occurs at pressing points, and can be fixed to the head 11. Preferably, the buffer 3 is made of urethane resin, for example. The thickness of the material of the buffer 3 may be appropriately determined in conformity with the material. In the case of the urethane resin in the embodiment, the thickness is about three millimeters.

In the embodiment of the present invention, a case where the buffer 3 is provided over the entire inner periphery of the outer layer 2 is described. The configuration is not limited thereto. For example, a configuration may be adopted where the buffer 3 may be provided on the inner periphery at positions which is around the temporal regions when the orthosis 1 is worn at the front and at which the pressers 24a and 24b are formed when the orthosis 1 is worn in a deviating manner, and the buffer 3 is not provided at the remaining part of the inner periphery.

### (Adjuster)

The adjuster 4 adjusts the adjustment portions 23a and 23b so that the minor axis of the substantially elliptic shape can correspond to the width of the head 11. The adjuster 4 performs adjustment so as to be close to or apart from in the minor axis direction of the substantially elliptic shape, and adjusts the positions of the adjustment portions 23a and 23b so that the adjustment portions 23a and 23b can be in contact with the proximity of the temporal regions of the wearer when the orthosis 1 is worn at the front.

In the embodiment of the present invention, the adjuster 4 is made up of the connecting member (belt) having non-expansibility or resistance to expansion. The connecting member is arranged so as to achieve connection over the opening of the outer layer 2. The adjuster 4 may be any member only if this adjuster does not expand or contract according to open and close of the outer layer 2 at the opposite ends on the opening. The adjuster 4 (connecting member) is formed so as not to interfere with induction of rotating the head 11.

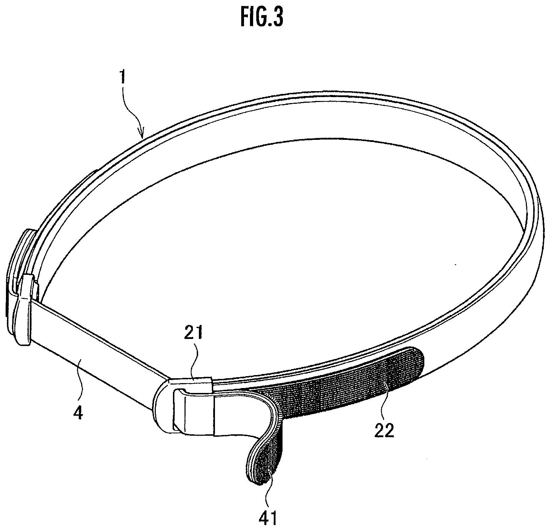

The connecting member of the adjuster 4 is, for example, surface fasteners (hook and loop fasteners). As shown in FIG. 3, one of a hook and a loop of each surface fastener is provided on the inner side of the connecting member. The other one of the hook and the loop of the surface fastener is provided on the outer side of the outer layer 2. More specifically, as shown in FIG. 1, the hooks 41 of the surface fasteners are fixed to the inner side (head side) of the adjuster 4 at its opposite ends with double-sided adhesive tape, adhesive or the like. Furthermore, the loops 22 of the surface fasteners are fixed to the outer side (the side opposite to the head) of the outer layer 2 in proximity to its opposite ends with double-sided adhesive tape, adhesive or the like. The loops 22 on the outer layer 2 are provided in a manner elongated in the circumferential direction, thereby allowing the opposite ends of the adjuster 4 to be fixed at predetermined positions. Consequently, the adjuster 4 can lock the opening of the outer layer 2 at an appropriate position, thus allowing the distance between the adjustment portions 23a and 23b to be adjusted.

Here, the outer layer 2 has elasticity. Consequently, the positions of the adjustment portions 23a and 23b are changed in a manner allowing the positions to be close to or apart from each other in the minor axis direction by bringing the opposite ends of the opening close to or apart from each other. The adjuster 4 locks the adjustment portions 23a and 23b at desired positions so that the positions of the adjustment portions 23a and 23b are not apart to exceed a predetermined width. The wearer wears the orthosis 1 in a state where the length of the minor axis of the orthosis 1 corresponds to the width of the head 11, and the adjuster 4 locks, thereby allowing the orthosis 1 not to be widened exceeding the distance of the width of the head 11 of the wearer. Consequently, when the orthosis is worn in a manner deviating from the positions, the pressers 24a and 24b are formed with the outer layer 2 being slightly expanded at the deviating positions. The formation can appropriately press the predetermined sites.

As described above, the adjuster 4 prevents the positions of the adjustment portions 23a and 23b from being apart exceeding the "predetermined width". Here, the "predetermined width" is larger than a value corresponding to the width of the head 11. The "predetermined width" is a width achieved by expanding the outer layer 2 when the orthosis 1 is worn with deviation, and is a width where the head 11 can be appropriately pressed at the pressers 24a and 24b.

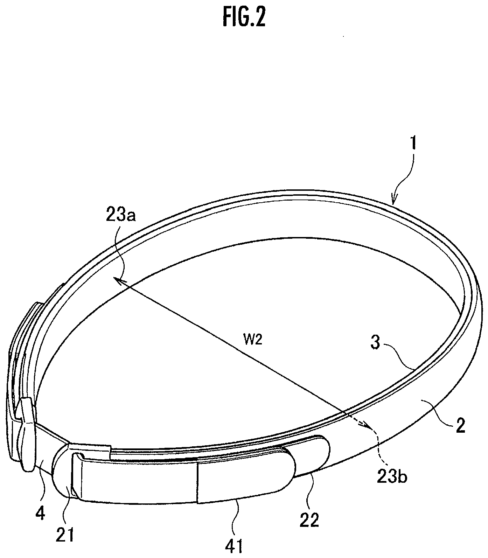

FIG. 1 shows a case where the adjustment portions 23a and 23b are provided apart from each other in the minor axis direction. FIG. 2 shows a case where the adjustment portions 23a and 23b are provided close to each other. The width W1 between the adjustment portions 23a and 23b in FIG. 1 is wider than the width W2 between the adjustment portions 23a and 23b in FIG. 2.

As to the adjustment portions 23a and 23b, the hooks 41 of the adjuster 4 can be connected at positions close to the ends of the outer layer 2 of the loops 22. Such connection reduces portions of the adjuster 4 where the belt and the outer layer 2 overlap with each other, and increases portions of the adjuster 4 where the belt and the outer layer 2 do not overlap with each other. In this case, as show in FIG. 1, while the opposite ends of the outer layer 2 become apart from each other, the adjustment portions 23a and 23b are also arranged apart from each other, thereby allowing the width W1 between the adjustment portions 23a and 23b to be widely fixed.

On the other hand, as to the adjustment portions 23a and 23b, the hooks 41 of the adjuster 4 can be connected at positions remote from the ends of the outer layer 2 of the loops 22. Such connection increases portions of the adjuster 4 where the belt and the outer layer 2 overlap with each other, and reduces a portion of the adjuster 4 where the belt and the outer layer 2 do not overlap with each other. In this case, as shown in FIG. 2, while the opposite ends of the outer layer 2 become close to each other, the adjustment portions 23a and 23b are also arranged close to each other, thereby allowing the width W2 between the adjustment portions 23a and 23b to be narrowly fixed.

In the embodiment of the present invention, the areas where the belt of the adjuster 4 and the outer layer 2 overlap with each other are adjusted, and fixed by the surface fasteners, thereby enabling the distance between the adjustment portions 23a and 23b to be appropriately adjusted. Thus, the width W of the orthosis 1, which is in close contact with the head 11, can be appropriately adjusted. Consequently, this single product can be adjusted to a wide variety and a large number of wearers.

In the embodiment of the present invention, the adjuster 4 is the connecting member (belt), which has non-expansibility or resistance to expansion. Consequently, this adjuster has a low rigidity and is easy to deform, but does not expand or contract according to open and close of the opposite ends of the outer layer 2. The outer layer 2 has elasticity, and includes an opening. Consequently, when the orthosis 1 is not worn, the orthosis 1 is deformed in a direction of closing the opening even with this orthosis being fixed by the belt. However, when the orthosis 1 is worn, adjustment and fixation are performed so that the width between the temporal regions of the head 11 can correspond to the width W between the adjustment portions 23a and 23b. Consequently, even when the orthosis 1 is rotated to deviate in the circumferential direction, the width W between the adjustment portions 23a and 23b does not become shorter than the width between the temporal regions of the head 11. While the width W between the adjustment portions 23a and 23b is slightly expanded by the elasticity of the outer layer 2, the width is locked by the adjuster 4 so as not to be largely expanded. Consequently, the orthosis 1 can apply appropriate pressures due to the elasticity of the outer layer 2 at the pressers 24a and 24b.

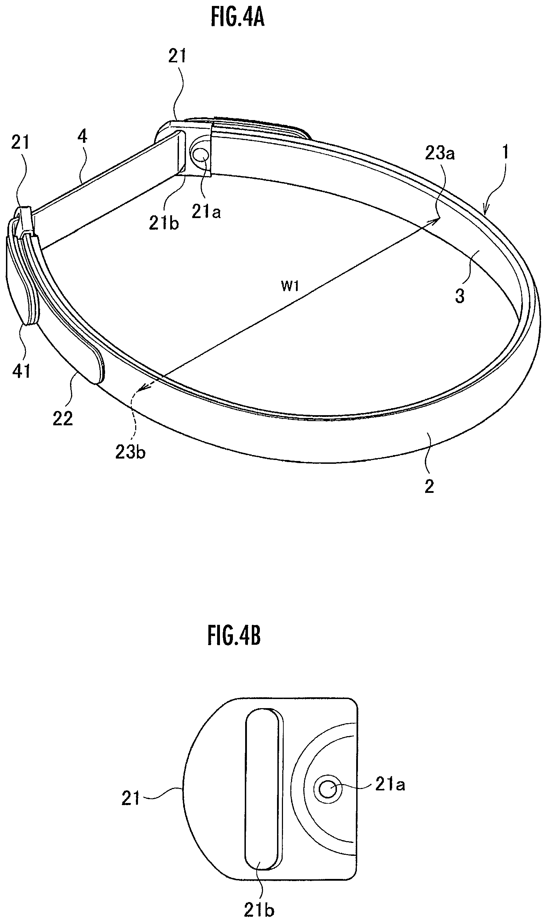

In the embodiment of the present invention, the outer layer 2 includes supporters 21 on the opposite sides of the opening of the outer layer 2 as shown in FIG.s 1 to 4. The supporters 21 may be provided at the opposite ends of the opening of the outer layer 2, and have a role as caps for covering the opposite ends. As shown in FIG.s 4A and 4B, each supporter 21 includes a screw fastener 21a and a hole 21b. Insertion of screws at the screw fasteners 21a allows the supporters 21 to be arranged so as to cover the respective ends of the outer layer 2. The hole 21b allows the connecting member to be inserted therethrough. According to the embodiment of the present invention, the connecting member is inserted through the hole 21b, and the surface fastener on the inner side of the connecting member is connected to the surface fastener on the outer side of the outer layer 2. Thus, a configuration where the surface fastener is resistant to disconnection is achieved. As shown in FIG. 4B, the hole 21b of the supporter 21 may be formed so as to have a small portion in the outer direction of the head, thereby allowing the connecting member of the adjuster 4 to be supported at the edge on the outer side of the hole as shown in FIG. 4A. Thus, a configuration where the surface fastener is further resistant to disconnection is achieved.

The opposite ends of the connecting member are formed to be thicker than the size of the hole, thereby allowing the connecting member to be resistant to getting out of the hole 21b, and enabling loss of the connecting member to be avoided.

### (Operation of orthosis)

Referring to FIG.s 5 to 7, an operation is described where after the wearer wears the orthosis 1 at the front, subsequent deviation causes the pressers 24a and 24b.

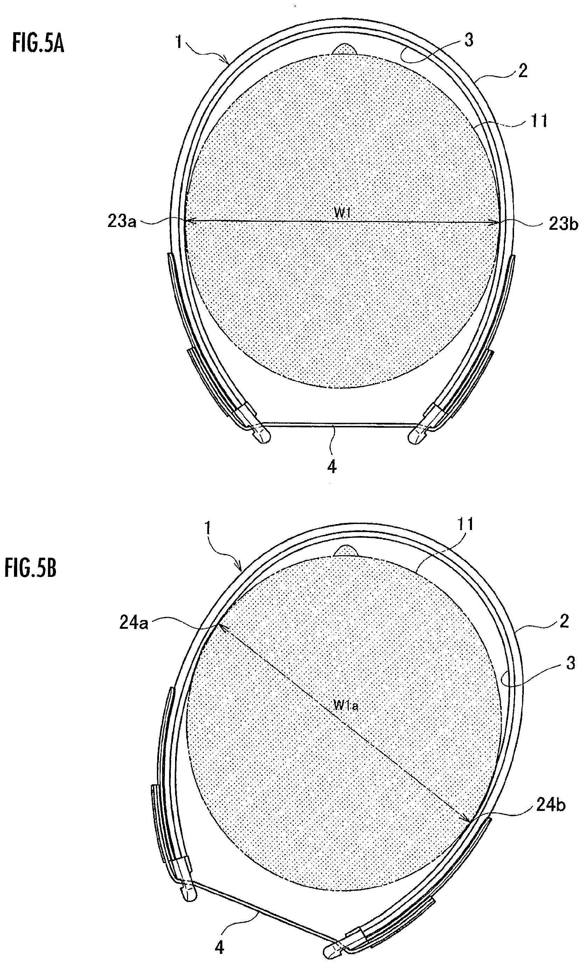

FIG. 5 is a top view of a state where a wearer having a wide head width wears the orthosis 1. As shown in FIG. 5A, when the wearer wears the orthosis 1 at the front and performs adjustment through the adjuster 4, the adjustment portions 23a and 23b of the orthosis 1 are in contact with the temporal regions of the wearer. The distance between the adjustment portions 23a and 23b is W1. The front and rear portions of the orthosis 1 are not in contact with the wearer or are in slight contact with the wearer.

The orthosis 1 fits the shape of the head and has a substantially elliptic shape. Consequently, the orthosis 1 can be rotated with the orthosis 1 being worn on the head 11. When the orthosis 1 is rotated in the direction toward a temporal region, the elasticity of the outer layer 2 exerts an effect which causes the shape of the head 11 and the shape of the orthosis 1 to deviate from each other owing to the rotation. As a result, as shown in FIG. 5B, for example, two pressers 24a and 24b are formed and forces are applied to the pressers 24a and 24b. The thus rotated orthosis 1 reaches a limit of deformation in a state of being in contact with the pressers 24a and 24b on the head 11, and becomes stationary while pressing the head 11.

At this time, the outer layer 2 of the orthosis 1 has elasticity. Consequently, the outer layer 2 is deformed outward at the pressers 24a and 24b, thereby causing the distance between the pressers 24a and 24b to be W1a (> W1). As shown in FIG. 5B, in a case where the buffer 3 is made of force-absorbable urethane, the buffer 3 (urethane) is compressed at the pressers 24a and 24b, and the shape is deformed. Here, locking is achieved by the adjuster 4 so that the orthosis 1 is not widely expanded in the minor axis direction of the substantially elliptic shape. Consequently, the distance between the pressers 24a and 24b cannot be expanded widely beyond the limit of deformation of the outer layer 2. Appropriate pressure at the pressers 24a and 24b can be achieved.

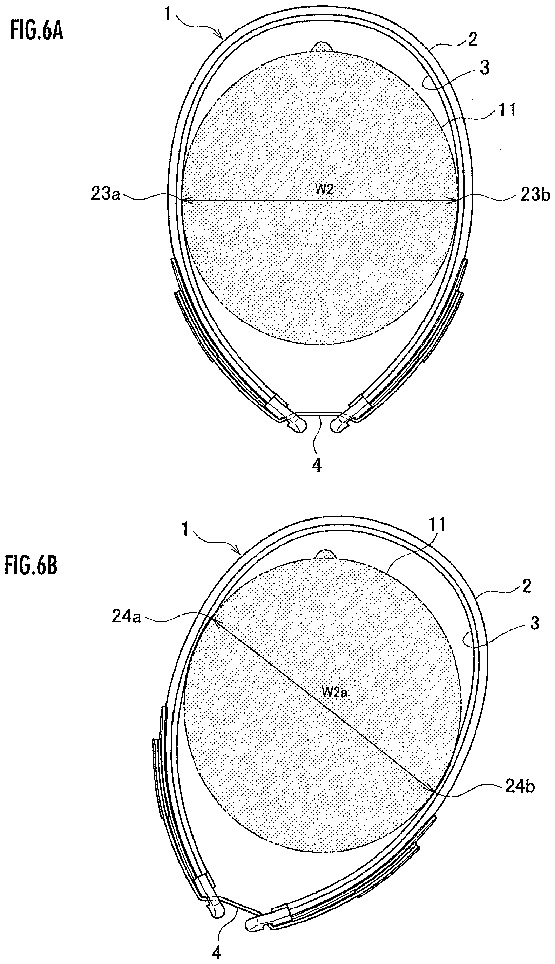

FIG. 6 is a top view of a state where a wearer having a narrow head width wears the orthosis 1. The outer layer 2 of the orthosis 1 has elasticity. Consequently, the outer layer 2 is deformed outward at the pressers 24a and 24b, thereby causing the distance between the pressers 24a and 24b to be W2a (> W2). As with FIG. 5, also in FIG. 6, locking is achieved by the adjuster 4 so that the orthosis 1 is not widely expanded in the minor axis direction of the substantially elliptic shape. Consequently, the distance between the pressers 24a and 24b cannot be expanded widely beyond the limit of deformation of the outer layer 2. Appropriate pressures at the pressers 24a and 24b can be achieved.

Thus, the orthosis 1 can press the predetermined sites on the head 11 while a patient can wear the orthosis 1 without this orthosis slipping down from the head 11.

Furthermore, referring to FIG. 7, the pressers 24a and 24b formed when the orthosis 1 is worn in a deviating manner are described. As shown in FIG. 7A, rotation of the orthosis 1 according to the embodiment of the present invention in the circumferential direction by a degree α causes the pressers 24a and 24b which receive stronger pressures than those before deviation, and allowance portions 5a and 5b which receive weaker pressures and have larger allowances than those before deviation.

It is herein assumed that the orthosis 1 has no elasticity. If the contour of the orthosis 1 overlaps the contour of the head 11 as shown in FIG. 7B, a distortion with a distance d is caused between the contour of the orthosis 1 and the contour of the head 11 at the presser 24a. However, in the embodiment of the present invention, the outer layer 2 of the orthosis 1 has elasticity. Consequently, the contour of the orthosis 1 fits the head 11. The outer layer 2 is expanded in conformity with the shape of the contact portion of the head 11, and the presser 24a is formed.

### (Pressing site of orthosis)

Referring to FIG.s 8 to 11, the positions and rotation pattern of the pressers 24a and 24b formed by the orthosis 1 according to the embodiment of the present invention are described. As shown in FIG.s 8 to 11, the orthosis 1 can induce each of rotations according to the positions where the pressers 24a and 24b are formed; the rotations include right and left (clockwise and counterclockwise) rotation, lateroversion rotation, anteroposterior rotation, and combined rotation thereof. In the embodiment of the present invention, the "lateroversion" indicates a state where the head is inclined to the right or left. The "anteroposterior" indicates a state where the head is inclined forward or backward. In FIG.s 8 to 11, each diagram A is a diagram of observing the wearer in a top view, each diagram B is a diagram of observing the wearer in a right view, and each diagram C is a diagram of observing the wearer in a left view. The positions shown in FIG.s 8 to 11 are only examples, and can be changed according to the symptom and state of each wearer.

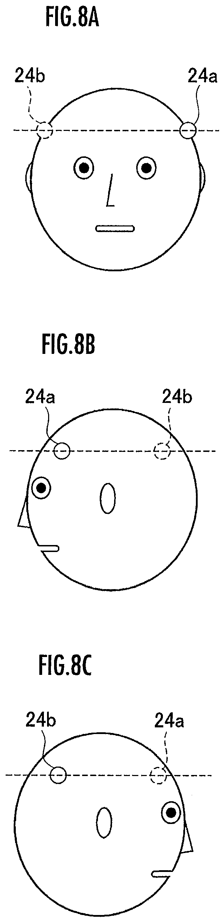

First, referring to FIG. 8, the clockwise and counterclockwise rotation pattern of the head 11 accompanying application of pressures to the front of a temporal region and the rear of the opposite temporal region is described. The wearer horizontally wears the orthosis 1 at a height around the temples of the head 11. The wearer performs adjustments through the adjuster 4 to cause the distance between the adjustment portions 23a and 23b to fit the head width. The wearer rotates the orthosis 1 in any of clockwise and counterclockwise directions to cause the adjustment portions 23a and 23b to coincide with the front of a temporal region and the rear of the opposite temporal region, thereby forming the pressers 24a and 24b at the front of the temporal region and the rear of the opposite temporal region. Thus, the rotations in the clockwise and counterclockwise directions can be induced. Therefore, for example, the symptom of spasmodic torticollis in the clockwise and counterclockwise directions can be alleviated. In the example of FIG. 8, the counterclockwise rotation is induced. Consequently, spasmodic torticollis in the clockwise direction can be corrected.

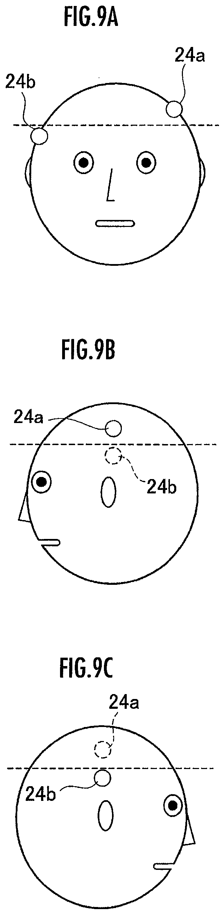

Next, referring to FIG. 9, the rotation pattern of the head 11 in the lateroversion direction accompanying application of pressures to the upper part of a temporal region and the lower part of the opposite temporal region is described. The wearer horizontally wears the orthosis 1 at a height around the temples of the head 11. The wearer performs adjustments through the adjuster 4 to cause the distance between the adjustment portions 23a and 23b to fit the head width. The wearer causes a portion of the orthosis 1 around a temporal region to deviate upward and causes the other portion to deviate downward to cause the adjustment portions 23a and 23b to coincide with the upper part of the temporal region and the lower part of the opposite temporal region, thereby forming the pressers 24a and 24b at the upper part of the temporal region and the lower part of the opposite temporal region. Thus, the rotation in the lateroversion direction can be induced. Therefore, for example, the symptom of spasmodic torticollis in the lateroversion direction can be alleviated. In the example of FIG. 9, the right lateroversion rotation is induced. Consequently, spasmodic torticollis in the left lateroversion direction can be corrected.

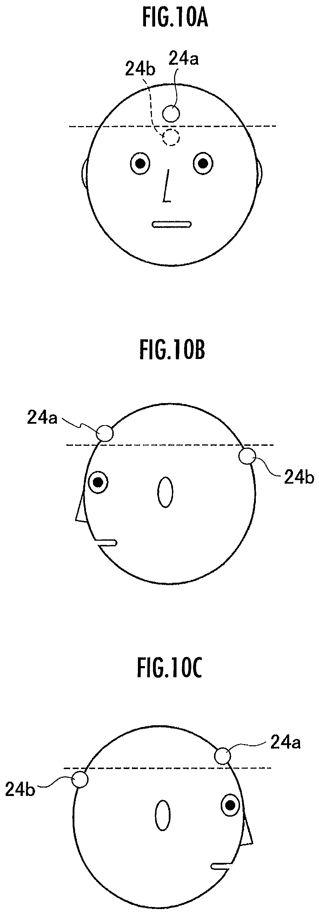

Next, referring to FIG. 10, the anteroposterior rotation pattern of the head 11 accompanying application of pressures to the upper part of the forehead and the lower part of the occiput is described. The wearer horizontally wears the orthosis 1 around the temples of the head 11. The wearer performs adjustments through the adjuster 4 to cause the distance between the adjustment portions 23a and 23b to fit the head width. The orthosis 1 is caused to deviate to the upper part of the forehead and the lower part of the occiput to cause the adjustment portions 23a and 23b to coincide with the upper part of the forehead and the lower part of the occiput, thereby forming the pressers 24a and 24b at the upper part of the forehead and the lower part of the occiput. Thus, the rotation in the anteroposterior direction can be induced. Therefore, for example, the symptom of spasmodic torticollis in the anteroposterior direction can be alleviated. In the example of FIG. 10, the rotation in the retroflexion direction is induced. Consequently, spasmodic torticollis in the anteflexion direction can be corrected.

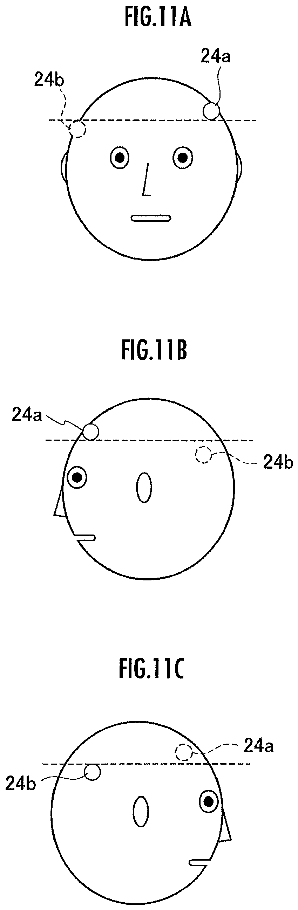

Next, referring to FIG. 11, a combined rotation pattern of the head 11 in the lateroversion and anteroposterior directions accompanying application of pressures to the upper left forehead and the lower right occiput is described. The wearer horizontally wears the orthosis 1 around the temples of the head 11. The wearer performs adjustments through the adjuster 4 to cause the distance between the adjustment portions 23a and 23b to fit the head width. The orthosis 1 is caused to deviate to cause the adjustment portions 23a and 23b to coincide with the upper left forehead and the lower right occiput, thereby forming the pressers 24a and 24b at the upper left forehead and the lower right occiput. In the example of FIG. 11, the rotation in the upper left direction is induced. Consequently, spasmodic torticollis in the lower right direction can be corrected.

The example shown in FIG. 11 is a combination of the rotation patterns described with reference to FIG.s 9 and 10. Such a combination of multiple patterns can support complex rotation patterns. Consequently, the orthosis 1 according to the embodiment of the present invention can induce rotations in all the directions.

### (First variation)

According to the embodiment of the present invention, the case has been described where the adjuster 4 is formed of the belt and is fixed to the outer layer by the surface fasteners, thereby locking the opening of the outer layer 2. However, the configuration is not limited thereto.

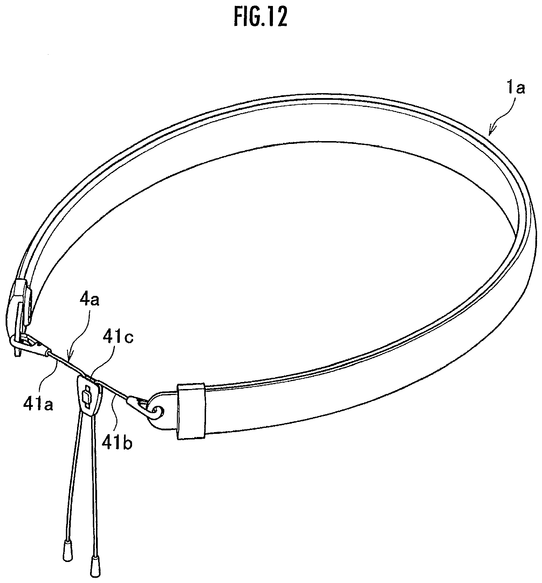

For example, as shown in FIG. 12, the adjuster 4a of the orthosis 1a comprises: a first connecting member 41a and a second connecting member 41b which are connected to the respective ends of the opening; and a fixing section (fixing fastener) 41c which adjusts the position at which the first connecting member 41a and the second connecting member 41b are connected each other and fixes the members.

In the example shown in FIG. 12, the first connecting member 41a and second connecting member 41b are string-like members having non-expansibility or resistance to expansion. The first connecting member 41a is connected to a hole provided for the outer layer 2 at the right end of the opening, and the second connecting member 41b is connected to a hole provided for the outer layer 2 at the left end of the opening. Furthermore, the first connecting member 41a and second connecting member 41b are fixed by the fixing section 41c, thereby allowing the opening of the outer layer 2 to be locked at a predetermined position.

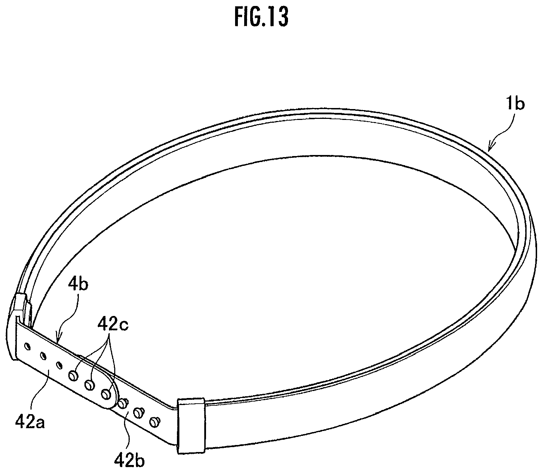

As shown in FIG. 13, the adjuster 4b of the orthosis 1b comprises: a first connecting member 42a and a second connecting member 42b which are connected to the respective ends of the opening; and a fixing section (fixing fastener) 42c which adjusts the position at which the first connecting member 42a and the second connecting member 42b are connected to each other and fixes the members.

In the example shown in FIG. 13, the first connecting member 42a and second connecting member 42b are belt-like members having non-expansibility or resistance to expansion. The second connecting member 42b is provided with multiple projections at predetermined intervals. The projections form a fixing section 42c. In the first connecting member 42a, multiple holes corresponding to the intervals and sizes of the protrusions of the second connecting member 42b are formed. The fixing section 42c of the protrusions of the second connecting member 42b are inserted into the holes of the first connecting member 42a, thereby allowing the opening of the outer layer 2 to be locked at a predetermined position.

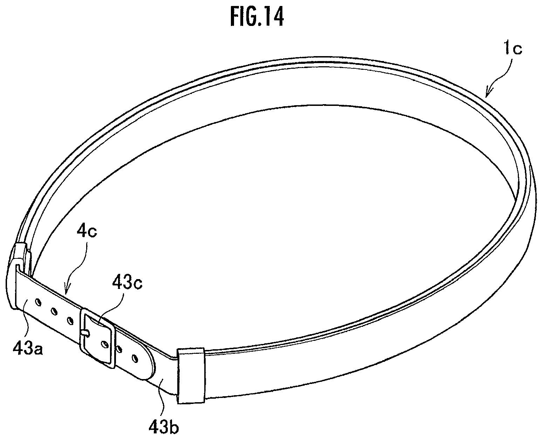

As shown in FIG. 14, the adjuster 4c of the orthosis 1c comprises: a first connecting member 43a and a second connecting member 43b which are connected to the respective ends of the opening; and a fixing section (fixing fastener) 43c which adjusts the position at which the first connecting member 43a and the second connecting member 43b are connected to each other and fixes the members.

In the example shown in FIG. 14, the first connecting member 43a and second connecting member 43b are belt-like members having non-expansibility or resistance to expansion. The second connecting member 43b is connected with the fixing section 43c which has a configuration similar to that of a buckle of a belt and includes a protrusion to be engaged into the hole. In the first connecting member 43a, multiple holes each corresponding to the size of the protrusion are formed. The fixing section 43c is inserted into the first connecting member 43a, and the protrusion of the fixing section 43c is engaged into a hole of the first connecting member 43a, thereby allowing the opening of the outer layer 2 to be locked at a predetermined position.

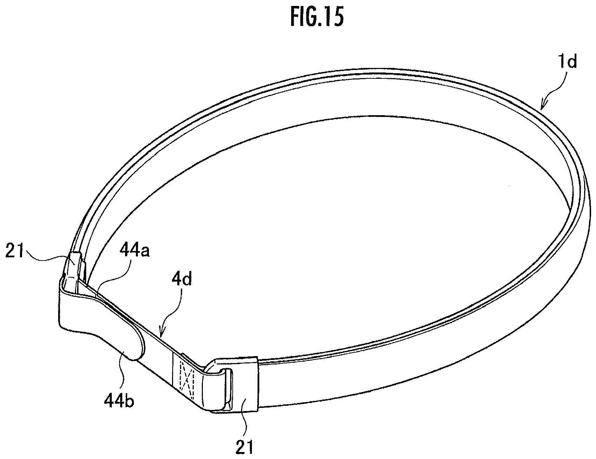

As shown in FIG. 15, an orthosis 1d comprising no surface fastener on an outer layer 2, and the adjuster 4d may be adopted. In the orthosis 1d shown in FIG. 15, the supporters 21 described in the embodiment of the present invention are formed at the opposite ends of the outer layer 2. One end of the belt described in the embodiment of the present invention is connected to one distal end of the outer layer 2. The belt is inserted through the hole of the supporter 21 and fixedly connected to the supporter 21 by sewing or the like. The other end of the belt is inserted into the hole of the supporter 21 at the end of the outer layer 2 where the belt is not connected, and then is folded back. Here, at a midway 44a and the distal end 44b on the external side (side opposite to the head) of the belt, surface fasteners are provided, thereby enabling the opening of the outer layer 2 to be locked at a predetermined position.

Thus, various patterns may be adopted as embodiments of the adjuster 4. Any implementation may be adopted only if the opening of the outer layer 2 can be locked at the predetermined position to form the pressers 24a and 24b.

### (Second variation)

Referring to FIG.s 16 and 17, orthoses 1e and 1f according to a second variation are described. The orthoses 1e and 1f according to the second variation are different from the orthosis 1 according to the embodiment of the present invention in that the orthoses comprise detachable sections. The detachable section is a portion which is detachably attached to the inner side of the buffer and is in contact with the head 11. The detachable section is formed of a member which transmits the pressure to the head 11 to the buffer. This configuration can exert advantageous effects similar to those in the case without attachment even when the detachable section is attached.

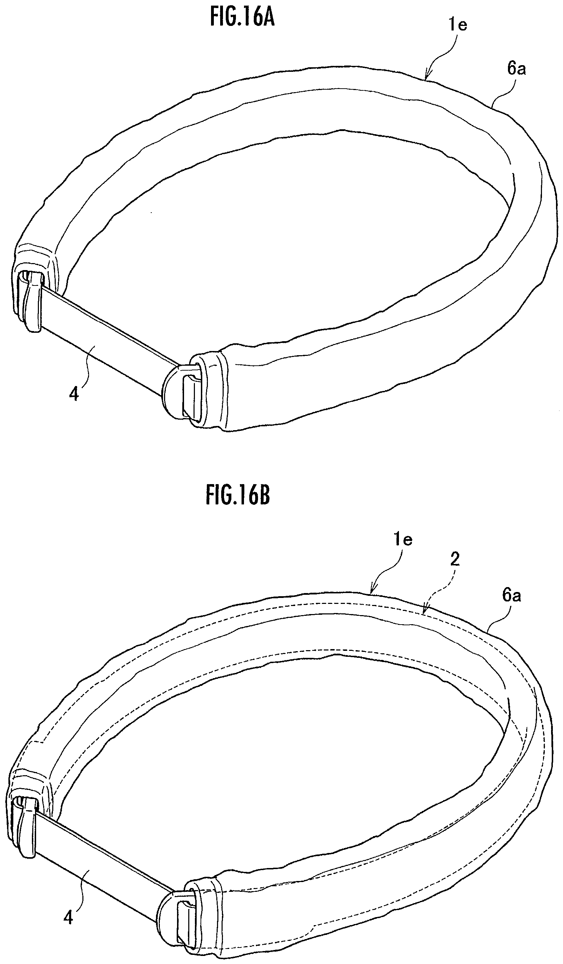

As to the orthosis 1e shown in FIG. 16, the detachable section 6a is formed to have a tubular form, thereby covering the outer layer 2 and the buffer 3 entirely. Thus, even if the outer layer 2 is shaped with metal, such as anodized aluminum, the outer layer 2 can be concealed with the detachable section 6a, which gives an impression as if it were bandanna. Consequently, the wearer can wear the orthosis 1e without concern for eyes of people therearound. Furthermore, the inner side of the buffer 3 is also covered with the detachable section 6a. Consequently, contamination due to contact of the orthosis 1e with the head 11 can be prevented from adhering to the buffer 3. The detachable section 6a can be attached and detached. Consequently, the wearer can cleanly wear the orthosis by washing and replacing the detachable section 6a.

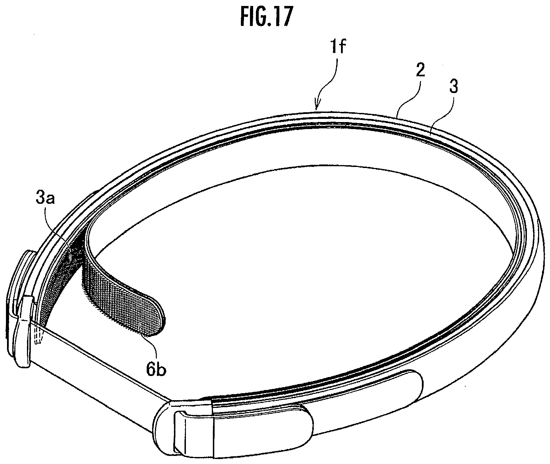

As to the orthosis If shown in FIG. 17, the detachable section 6b is formed of a surface fastener. The loop 3a of the surface fastener is fixed onto the inner side of the buffer 3 with double-sided adhesive tape or adhesive, and the detachable section 6b is formed of a belt to which a hook is attached. Such a detachable section 6b can be attached and detached. Consequently, the wearer can cleanly wear the orthosis by washing and replacing.

### (Other embodiments)

The description has thus been made according to the embodiments of the present invention, the first variation, and the second variation. The statements and diagrams which constitute a part of this disclosure should not be construed to limit the present invention. According to this disclosure, various alternative embodiments, exemplary embodiments and operational techniques are clarified for those skilled in the art.

For example, according to each embodiment of the present invention, the description has been made on the cases where the number of pressing points of hanger reflex is two. Alternatively, the number may be one, or three or more.

According to the embodiments of the present invention, the description has been made on the cases where the outer layer 2 has the opening to have substantially C-shape. The technique is also applicable to cases where no opening is provided.

It is a matter of course that the present invention encompasses various embodiments which are not described here. Consequently, the technical scope of the present invention is determined only by matters to define the invention according to the claims which is appropriate based on the above description.

### Reference Signs List

- 1: Orthosis
- 2: Outer layer
- 3: Buffer
- 4: Adjuster
- 5a, 5b: Allowance portions
- 11: Head
- 21: Supporter
- 21a: Screw fastener
- 21b: Hole
- 22: Loop
- 23a, 23b: Adjustment portions
- 24a, 24b: Pressers
- 41: Hook

## Claims

1. An orthosis (1), comprising:
an outer layer (2) which maintains a substantially elliptic shape; and
a buffer (3) which is provided on an inner periphery of the outer layer (2), and buffers a predetermined site on a head,
**characterized in that** the outer layer (2) comprises adjustment portions (23a, 23b) which can be close to or apart from each other in a direction of the minor axis of the substantially elliptic shape, and
further comprises an adjuster (4) which is adapted to adjust the adjustment portions (23a, 23b) so as to allow the minor axis of the substantially elliptic shape to have a length corresponding to the width of the head (11),
the adjuster (4) is formed of a connecting member having non-expansibility or resistance to expansion,
the outer layer has a substantially C-shape formed by providing an opening at a front or rear in a direction of a major axis of the substantially elliptic shape, and
the connecting member is arranged so as to connect the opening, and
when the orthosis (1) is worn in a state deviating from a state where the minor axis of the substantially elliptic shape of the outer layer (2) corresponds to the width of the head (11), the outer layer (2) is expanded in conformity with the shape of a contact portion of the head (11), and due to elastic deformation, pressers (24a, 24b) which press the predetermined site on the head (11) are formed to induce a rotational movement of the head (11).

2. The orthosis (1) according to claim 1,
wherein a ratio of change in the substantially elliptic shape in the direction of the minor axis due to adjustment of the adjuster (4) is larger than a ratio of change in the substantially elliptic shape in the direction of the major axis.

3. The orthosis (1) according to claim 1,
wherein when the adjustment portions (23a, 23b) are adjusted by the adjuster (4) so that the length of the minor axis of the substantially elliptic shape can be adapted to correspond to the width of the head (11), the connecting member is arranged at a position which does not press the head (11).

4. The orthosis (1) according to claim 1,
wherein the connecting member has a belt shape, and one of a hook (41) and a loop (22) of a surface fastener is provided on an inner side of the connecting member, and
another one of the hook (41) and the loop (22) of the surface fastener is provided on an outer side of the outer layer (2).

5. The orthosis (1) according to claim 4, further comprising
supporters (21) each having a hole through which the connecting member is to be inserted are provided at opposite ends of the opening of the outer layer (2),
wherein the connecting member is adapted to be inserted through the hole, and the surface fastener on the inner side of the connecting member is connected to the surface fastener on the outer side of the outer layer (2).

6. The orthosis (1) according to claim 1,
wherein the connecting member is formed of
a first connecting member and a second connecting member which are respectively connected to opposite ends of the opening, and
a fixing section (41c, 42c, 43c) which adjusts and fixes a position where the first connecting member and the second connecting member are connected to each other, and
according to the position of fixation by the fixing section (41c, 42c, 43c), lengths from the opposite ends of the opening to the fixing section (41c, 42c, 43c) are adjusted to adjust the adjustment portions (23a, 23b) so that the length of the minor axis of the substantially elliptic shape can be adapted to correspond to the width of the head (11).

7. The orthosis (1) according to claim 1, further comprising
a detachable section (6a, 6b) which is detachably attached to an inner side of the buffer (3) and is in contact with the head (11),
wherein the detachable section (6a, 6b) is formed of a member which is adapted to transmit pressure on the head (11) to the buffer (3).

## Patentansprüche

1. Orthese (1), umfassend:
eine äußere Schicht (2), welche eine im Wesentlichen elliptische Form beibehält; und
einen Puffer (3), welcher an einem inneren Umfang der äußeren Schicht (2) bereitgestellt ist, und eine vorbestimmte Seite an einem Kopf dämpft,
**dadurch gekennzeichnet, dass**
die äußere Schicht (2) Einstellabschnitte (23a, 23b) umfasst, welche voneinander in einer Richtung der Nebenachse der im Wesentlichen elliptischen Form angenähert oder entfernt sein können, und
ferner eine Einstelleinheit (4) umfasst, dazu eingerichtet ist, die Einstellabschnitte (23a, 23b) einzustellen, um der Nebenachse der im Wesentlichen elliptischen Form zu erlauben, eine Länge aufzuweisen,
welche der Breite des Kopfs (11) entspricht,
die Einstelleinheit (4) aus einem Verbindungselement gebildet ist,
welches eine Nicht-Dehnbarkeit oder eine Widerstandsfähigkeit gegenüber Dehnung aufweist,
die äußere Schicht im Wesentlichen eine C-Form aufweist, welche durch Bereitstellen einer Öffnung an einer Vorderseite oder einer Rückseite in einer Richtung einer Hauptachse der im Wesentlichen elliptischen Form gebildet ist, und
das Verbindungselement angeordnet ist, die Öffnung zu verbinden, und
wenn die Ortthese (1) in einem Zustand getragen ist, welcher von einem Zustand abweicht, in welchem die Nebenachse der im Wesentlichen elliptischen Form der äußeren Schicht (2) der Breite des Kopfs (11) entspricht, sich die äußere Schicht (2) im Einklang mit der Form eines Kontaktabschnitts des Kopfs (11) erweitert, und, aufgrund einer elastischen Deformation, Druckelemente (24a, 24b), welche die vorbestimmte Seite des Kopfs (11) drücken, gebildet sind, um eine rotatorische Bewegung des Kopfs (11) einzuleiten.

2. Orthese (1) nach Anspruch 1,
wobei ein Verhältnis einer Änderung in der im Wesentlichen elliptischen Form in der Richtung der Nebenachse aufgrund einer Einstellung der Einstelleinheit (4) größer als ein Verhältnis einer Änderung in der im Wesentlichen elliptischen Form in der Richtung der Hauptachse ist.

3. Orthese (1) nach Anspruch 1,
wobei, wenn die Einstellabschnitte (23a, 23b) durch die Einstelleinheit (4) eingestellt werden, so dass die Länge der Nebenachse der im Wesentlichen elliptischen Form dazu angepasst werden kann, der Breite des Kopfs (11) zu entsprechen, das Verbindungselement an einer Position angeordnet ist, welche den Kopf (11) nicht drückt.

4. Orthese (1) nach Anspruch 1,
wobei das Verbindungselement eine Riemenform aufweist, und eines aus einem Haken (41) und einer Schlaufe (22) einer Oberflächenbefestigung an einer inneren Seite des Verbindungselements bereitgestellt ist, und
ein anderes aus dem Haken (41) und der Schlaufe (22) der Oberflächenbefestigung an einer äußeren Seite der äußeren Schicht (2) bereitgestellt ist.

5. Orthese (1) nach Anspruch 4, ferner umfassend Halterungselemente (21), welche jeweils ein Loch aufweisen, durch welches das Verbindungselement einzusetzen ist, welche an gegenüberliegenden Enden der Öffnung der äußeren Schicht (2) bereitgestellt sind,
wobei das Verbindungselement dazu eingerichtet ist, durch das Loch eingesetzt zu werden, und die Oberflächenbefestigung an der inneren Seite des Verbindungselements mit der Oberflächenbefestigung an der äußeren Seite der äußeren Schicht (2) verbunden ist.

6. Orthese (1) nach Anspruch 1,
wobei das Verbindungselement gebildet ist aus einem ersten Verbindungselement und einem zweiten Verbindungselement, welche jeweils mit gegenüberliegenden Enden der Öffnung verbunden sind, und
einem Befestigungsabschnitt (41c, 42c, 43c), welcher eine Position einstellt und fixiert, in welcher das erste Verbindungselement und das zweite Verbindungselement miteinander verbunden sind, und gemäß der Position einer Fixierung durch den Befestigungsabschnitt (41c, 42c, 43c) Längen von den gegenüberliegenden Enden der Öffnung zu dem Befestigungsabschnitt (41c, 42c, 43c) eingestellt werden, um die Einstellabschnitte (23a, 23b) derart einzustellen, dass die Länge der Nebenachse der im Wesentlichen elliptischen Form angepasst werden kann, der Breite des Kopfs (11) zu entsprechen.

7. Orthese (1) nach Anspruch 1, ferner umfassend einen lösbaren Abschnitt (6a, 6b), welcher an einer inneren Seite des Puffers (3) und in Kontakt mit dem Kopf (11) lösbar angebracht ist, wobei der lösbare Abschnitt (6a, 6b) aus einem Element gebildet ist, welches dazu eingerichtet ist, einen Druck auf den Kopf (11) an den Puffer (3) zu übertragen.

## Revendications

1. Orthèse (1), comprenant :
une couche extérieure (2) qui conserve une forme sensiblement elliptique ; et
un amortisseur (3) qui est prévu sur une périphérie intérieure de la couche extérieure (2) et amortit un site prédéterminé sur une tête,
**caractérisé en ce que** la couche extérieure (2) comprend des parties de réglage (23a, 23b) qui peuvent être proches ou espacées l'une de l'autre dans une direction du petit axe de la forme sensiblement elliptique, et
comprend en outre un élément de réglage (4) qui est adapté pour régler les parties de réglage (23a, 23b) de façon à permettre au petit axe de la forme sensiblement elliptique d'avoir une longueur correspondant à la largeur de la tête (11),
l'élément de réglage (4) est constitué d'un élément de liaison présentant une non-expansibilité ou une résistance à l'expansion,
la couche extérieure a une forme sensiblement en C formée en prévoyant une ouverture à l'avant ou à l'arrière dans une direction d'un grand axe de la forme sensiblement elliptique, et
l'élément de liaison est agencé de façon à être lié à l'ouverture, et
lorsque l'orthèse (1) est portée dans un état s'éloignant d'un état dans lequel le petit axe de la forme sensiblement elliptique de la couche extérieure (2) correspond à la largeur de la tête (11), la couche extérieure (2) est expansée conformément à la forme d'une partie de contact de la tête (11) et, sous l'effet d'une déformation élastique, des éléments de compression (24a, 24b) qui compriment le site prédéterminé sur la tête (11) sont formés pour induire un mouvement de rotation de la tête (11).

2. Orthèse (1) selon la revendication 1,
dans laquelle un rapport de changement de la forme sensiblement elliptique dans la direction du petit axe du fait du réglage de l'élément de réglage (4) est supérieur à un rapport de changement de la forme sensiblement elliptique dans la direction du grand axe.

3. Orthèse (1) selon la revendication 1,
dans laquelle, lorsque les parties de réglage (23a, 23b) sont réglées par l'élément de réglage (4) afin que la longueur du petit axe de la forme sensiblement elliptique puisse être adaptée pour correspondre à la largeur de la tête (11), l'élément de liaison est agencé dans une position qui ne comprime pas la tête (11).

4. Orthèse (1) selon la revendication 1,
dans laquelle l'élément de liaison a une forme de courroie, et un d'un crochet (41) et d'une boucle (22) d'un dispositif de fixation de surface est prévu sur un côté intérieur de l'élément de liaison, et
un autre du crochet (41) et de la boucle (22) du dispositif de fixation de surface est prévu sur un côté extérieur de la couche extérieure (2).

5. Orthèse (1) selon la revendication 4, comprenant en outre
des éléments de support (21) ayant chacun un orifice à travers lequel l'élément de liaison doit être inséré, qui sont prévus à des extrémités opposées de l'ouverture de la couche extérieure (2),
dans laquelle l'élément de liaison est adapté pour être inséré à travers l'orifice, et le dispositif de fixation de surface sur le côté intérieur de l'élément de liaison est relié au dispositif de fixation de surface sur le côté extérieur de la couche extérieure (2).

6. Orthèse (1) selon la revendication 1,
dans laquelle l'élément de liaison est constitué
d'un premier élément de liaison et d'un second élément de liaison qui sont respectivement reliés aux extrémités opposées de l'ouverture, et
d'une section de fixation (41c, 42c, 43c) qui règle et fixe une position dans laquelle le premier élément de liaison et le second élément de liaison sont reliés l'un à l'autre,
et
selon la position de fixation par la section de fixation (41c, 42c, 43c), les longueurs par rapport aux extrémités opposées de l'ouverture à la section de fixation (41c, 42c, 43c) sont réglées pour régler les parties de réglage (23a, 23b) afin que la longueur du petit axe de la forme sensiblement elliptique puisse être adaptée pour correspondre à la largeur de la tête (11).

7. Orthèse (1) selon la revendication 1, comprenant en outre
une section amovible (6a, 6b) qui est fixée de manière amovible à un côté intérieur de l'amortisseur (3) et est en contact avec la tête (11),
dans laquelle la section amovible (6a, 6b) est constituée d'un élément qui est adapté pour transmettre une pression sur la tête (11) à l'amortisseur (3).
